# EUROPÄISCHE PATENTSCHRIFT

(11) **EP 0 655 135 B1**
(45) Veröffentlichungstag und Bekanntmachung des Hinweises auf die Patenterteilung: **24.09.2003**
(21) Anmeldenummer: 94916122.8
(22) Anmeldetag: 07.06.1994
(51) Int. Cl.: G01N 33/48, C12Q 1/00

(54) **ZUFALLSERZEUGUNG UND BIO-SPEZIFISCHE AUSWAHL CHEMISCHER VERBINDUNGEN**
RANDOM GENERATION AND BIO-SPECIFIC SELECTION OF CHEMICAL COMPOUNDS
GENERATION ALEATOIRE ET SELECTION BIO-SPECIFIQUE DE COMPOSES CHIMIQUES

(30) Priorität: 16.06.1993 CH 179093
(43) Veröffentlichungstag der Anmeldung: 31.05.1995
(73) Patentinhaber: Bürgisser, Ernst, 4310 Rheinfelden (CH); Folkers, Gerd Peter, 8050 Zürich (CH)
(72) Erfinder: BÜRGISSER, Ernst, CH-4314 Zeiningen (CH); FOLKER, Gerd Peter, Prof. Dr., CH-8050 Zürich (CH)
(74) Vertreter: Frei, Alexandra Sarah
(86) Internationale Anmeldenummer: CH9400109
(87) Internationale Veröffentlichungsnummer: WO94029719

(56) Entgegenhaltungen:
- NATURE., Bd.354, Nr.6348, 7. November 1991, LONDON GB Seiten 82 - 84 KIT S. LAM ET AL. 'A new type of synthetic peptide library for identifying ligand-binding activity' in der Anmeldung erwähnt
- NATURE., Bd.354, Nr.6348, 7. November 1991, LONDON GB Seiten 84 - 86 RICHARD A. HOUGHTEN ET AL. 'Generation and use of synthetic peptide combinatorial libraries for basic research and drug discovery' in der Anmeldung erwähnt
- BIOORGANIC & MEDICINAL CHEMISTRY LETTERS, Bd.3, Nr.3, März 1993, OXFORD, GB Seiten 419 - 424 KIT S. LAM ET AL. 'The Chemical Synthesis of Large Random Peptide Libraries and their Use for the Discovery of Ligand for Macromolecular Acceptors'
- ANNALES DE BIOLOGIE CLINIQUE, Bd.49, Nr.4, April 1991, PARIS, FR Seiten 231 - 242 R.H. MELOEN ET AL. 'The use of peptides to reconstruct conformational determinants; a brief review'
- Dissertation ETH Nr. 9495 der ETH Zürich, Wolf-Peter Scheve (1991)
- Vorversion und Titelblatt der Dissertation ETH Nr. 13592 der ETH Zürich, Ulrich Kessler (2000)
- New J. Chem., Bd.16, S.233-242, (1992)
- Organic Preparations and Procedures International, Bd.3, S.685-711, (1971)
- Chem. Soc. Rev., Bd.20, S.1-47, (1991)

## Beschreibung

Die Erfindung liegt auf dem Gebiet der Synthese und Charakterisierung chemischer Verbindungen und betrifft ein Verfahren zur Zufalls-Synthese chemischer Verbindungen und zur spezifischen Prüfens solcher chemischer Verbindungen gemäss biologischer Testmethoden und deren Charakterisierung gemäss physikalischer, chemischer Analysemethoden.

Auf der Suche nach neuen pharmakologischen Wirkstoffen bedient sich die Pharma- und Agroforschung u.a. dem "Random Screening", d.h. es werden möglichst viele Reinsubstanzen und Naturextrakte gemäss dem Zufallsprinzip hinsichtlich ihrer möglichen Wirkung an spezifischen biologischen Testsystemen, wie beispielsweise an in vitro-Assaysystemen geprüft.

Das Random Screening beruht somit auf höchst effektiven, gemäss dem "Schloss und Schlüssel-Prinzip" arbeitenden Kausalzusammenhängen zwischen vorhandenen Agenzien und bestimmten, wohldefinierten in vitro-Assaysystemen. Sie sind sehr selektiv und vermögen kleinste Unterschiede der Molekülstruktur der in gegenseitigem Kontakt stehenden Partner zu unterscheiden. Dementsprechend richtet sich die Wahl solcher biologischen Testsysteme in erster Linie nach den spezifischen Indikationsgebieten, für welche eine neue Leitsubstanz gefunden werden soll.

Ein Nachteil des gebräuchlichen Screenings von Reinsubstanzen ist die Tatsache dass damit a priori keine neuen Verbindungen gefunden werden können, sondern lediglich eine unbekannte Eigenschaft entdeckt werden kann. Im Weiteren können geringste Verunreinigungen, welche mit üblichen chemischen Analysemethoden kaum erfasst werden können, eine biologische Antwort hervorrufen, welche dann irrtümlicherweise der Testsubstanz zugeschrieben wird. Ausserdem ist die molekulare Vielfalt von bestehenden chemischen Substanzansammlungen eher gering und die Anzahl verschiedener Substanzen limitiert.

Eine weitere, häufig benutzte Quelle für das Random-Screening sind Naturextrakte aus Pflanzen, Tieren und biotechnologischen Prozessen. Die Nachteile solcher Extrakte ist die Tatsache, dass bioaktive Komponenten oft hochmolekulare, polymere Moleküle wie z.B. Eiweisse sind, die als Medikamente oder Pflanzenschutzmittel aus produktions- und anwendungsbedingten Gründen eher nicht erwünscht sind.

Eine dritte, in der Praxis benutzte Quelle für das Screening, sind Peptide, welche sich chemisch-synthetisch oder mittels molekularbiologischen Methoden herstellen lassen. Dank ihrer Eigenschaft als linear aufgebaute Ketten aus definierten Bausteinen (Arninosäuren) lässt sich eine nahezu unendlich grosse Anzahl verschiedener Moleküle synthetisieren, welche einem Screening zugeführt werden können. Dazu wurden spezielle Strategien entwickelt (siehe Ref. 1) Houghten et al, Ref. 2) Lam et al). Obwohl in der Natur häufig Peptide als Botenstoffe (z.B. Hormone, Neurotransmitter) oder als Toxine (z.B. Schlangengifte) etc. benutzt werden, sind Eiweisse (Peptide und Proteine) als Wirkstoff in der Pharmazie nur bedingt von Interesse und werden wenn möglich durch nicht-peptidische Molekülstrukturen ersetzt (sog. Peptidomimetica). Die Gründe dafür liegen bei der schlechten oralen Aufnahme, sowie bei der raschen in-vivo Metabolisierung der Peptide.

Eine vierte Möglichkeit zur Erzeugung und Nutzung von molekularer Vielfalt ist ein zum oben beschriebenen Strategie der "Combinatorial Peptide Libraries" analoges Verfahren, wonach aber nicht-poymere organische Verbindungen synthetisiert werden. Obwohl damit eine grosse chemische Vielfalt erzeugt werden kann, handelt es sich dennoch um prospektiv geplante Synthesen, welche grundsätzlich keine Zufallsprodukte erzeugen.

Die Dissertation ETH Nr. 9495, der ETH Zürich, Scheve (1991) beschreibt der Effekt von Bestrahlung auf organische Moleküle. New J. Chem, Bd. 16, S 233-242 (1992) beschreibt die Aktivierung einer Reihe von bekonnten organischen Reaktionen durch Bestrahlung mit Mikrowellen.

Aus der vorangegangen Aufzählung geht hervor, dass es wünschenswert wäre, ein Verfahren zur Erzeugung chemischer Vielfalt aufzuzeigen, welches die beschriebenen Nachteile eliminiert, sowie effizient und damit kostengünstig durchgeführt werden kann.

Die Aufgabe der vorliegenden Erfindung ist es, ein Verfahren zur zufälligen Erzeugung und spezifischen Auswahl chemischer Verbindungen aufzuzeigen. Dieses Verfahren soll eine Zufallschemie ermöglichen. Es sollen bekannte, kombinatorisch gemischte Edukte zur Reaktion gebracht werden, wobei unter anderem bisher unbekannte Zufalls-Produkte entstehen, welche anschliessend biologischen Selektionsmethoden zugeführt und hinsichtlich einer möglichen Biospezifität untersucht werden. Aus den positiv reagierenden Cocktails C' lassen sich die biologisch aktiven Komponenten isolieren und deren chemische Strukturen identifizieren.

Diese Aufgabe wird durch die in den Patentansprüchen definierte Erfindung gelöst.

Die der Erfindung zugrundeliegende Idee besteht darin, aus einer relativ kleinen Zahl chemisch reiner Verbindungen eine grosse Zahl von kombinatorischen Mischungen herzustellen (Edukt-Cocktails C) um sie danach gewissen reaktionsvermittelnden Bedingungen auszusetzen, mit der Absicht neue, a priori unbekannte chemische Verbindungen zu erhalten. Die auf diese Weise entstandenen Produkte-Cocktails (C) werden sodann geeigneten biologischen Tests unterworfen, wobei im Falle einer positiven Reaktion die aktiven Substanzen isoliert und deren Molekülstrukturen aufgeklärt werden. Die Grundidee liegt in der Kombination einer Zufallschemie mit biologischen Selektionsverfahren, was in Analogie zur natürlichen Evolution mit Mutation und Selektion steht. Im Gegensatz zum bekannten Verfahren der "Combinatorial Peptide Libraries" von Houghten et al (Ref. 1) werden mit der beschriebenen Methode keine Polymere mit voraussagbarer Struktur gebildet, sondern unvorhersehbare Zufallsprodukte erzeugt, wobei eine grosse Wahrscheinlichkeit besteht, dass es sich dabei um bisher in der Literatur unbekannte Verbindungen handeln wird.

Anhand den nachfolgend beschriebenen Schritten wird das erfindungsgemässe Verfahren erläutert:

Schritt 1: Bekannte Ausgangssubstanzen werden zu einem Cocktail (C) gemischt. Als Ausgangssubstanzen kommen grundsätzlich alle löslichen chemischen Verbindungen in Betracht. Aus praktischen und technischen Gründen werden vorteilhafterweise bekannte, kostengünstige, niedermolekulare organische Verbindungen im Molekulargewichtsbereich bis ca.1000 Formelmasse verwendet Dabei ist es unerheblich, ob die Ausgangssubstanzen eine biologische Wirkung haben oder nicht. Obwohl eine gewisse Auswahl an Ausgangssubstanzen getroffen werden kann, darf aber beim Herstellen der Edukte-Cocktails auch das Zufallsprinzip spielen.

Im Prinzip lässt sich auf diese Weise eine beliebige Anzahl von Reinsubstanzen in jedem denkbaren Konzentrationsverhätnis zu einem Ausgangs-Cocktail C mischen. Durch systematisches Kombinieren von zwei oder mehreren Reinsubstanzen lässt sich aus einer Sammlung von relativ wenig Verbindungen eine grosse Anzahl Cocktails C herstellen. Dabei beeinflusst die Wahl des verwendeten Lösungsmittels (zB. Wasser oder organische Lösungsmittel wie Alkohol, Benzol, DMSO etc.) wesentlich den Reaktionsmechanismus. Ausserdem kann das Lösungsmittel bezw. das Lösungsmittelgemisch selbst an der chemischen Reaktion teilnehmen, womit auch das Lösungsmittel einen variierbaren Verfahrensparameter darstellt und somit einer Ausgangssubstanz gleichkommt.

Schritt 2: Die Cocktails C zur Initialisierung der Zufallschemie werden bestimmten chemischen und/oder physikalischen Bedingungen ausgesetzt, sodass neue, a priori unbekannte chemische Verbindungen als Produkte entstehen. Die physikalischen Bedingengen sind Bestrahlungen (ionisierende Strahlung, Neutronenbeschuss, Licht, UV, Mikrowelle, etc.). Als chemische Reaktionsvermittler bzw. Reaktionsverhinderer eignen sich beispielsweise pH-Wert, lonenstärke, Crosslinker und Radikalfänger um allfälliges übermässiges Reagieren zu drosseln. Die Zufallschemie geht hierbei streng nach deterministischen Prinzipien vor und lässt sich bei Kenntnis aller Systemparameter grundsätzlich reproduzieren.

Verfahrenstechnisch werden hierbei systematisch Reaktionsparameter wie beispielsweise Konzentration der Edukte, Temperaturrampen etc. variiert oder konstant gehalten. Diese Variation und Kombination lässt ein sehr breites Spektrum für die gewünschte Zufallschemie zu, sodass mit an Sicherheit grenzender Wahrscheinlichkeit bis anhin unbekannte biologisch wirksame Strukturen gebildet werden. Dem Fachmann stehen hierzu bei Kenntnis dieser Erfindung eine grosse Anzahl von Möglichkeiten offen.

Schritt 3: In diesem Verfahrensschritt werden aus der grossen Anzahl zufällig neu entstandener, chemischer Verbindungen biologisch aktive Komponenten heraus selektioniert. Hierfür bedient man sich biologischer Testsysteme B. Neben konventionellen Bioassays lassen sich auch Biosensoren einsetzen. Bioassay-Systeme arbeiten gemäss dem "Schloss und Schlüssel-Prinzip" und sind sehr empfindlich für kleinste Unterschiede in der Molekülstruktur der neu entstandenen chemischen Verbindungen. Die Wahl der biologischen Testsysteme B richtet sich in erster Linie nach dem Indikationsgebiet, für welches eine neue Leitsubstanz gesucht wird. Als solche Screening-Assaysysteme kommen beispielsweise in Frage: Rezeptor-Bindungsassays, Immunologische Testverfahren, Enzymtests, funktionelle Tests an ganzen Organismen, an Organen, an Geweben, auf zellulärer und subzellulärer Ebene, sowie Untersuchung der Wirkung auf die Erbsubstanz (DNA und RNA). Nur in einer beschränkten Anzahl von Reaktions-Cocktails C' wird man eine biologische Wirkung finden, und nur solche "Hits" werden dem nächsten Schritt zugeführt.

Schritt 4: Mit nicht-destruktiven Methoden (z.B. Chromatographie) wird der Reaktionscocktail C aufgetrennt und die einzelnen Fraktionen werden hinsichtlich der biologischen Wirkung mittels den im Schritt 3 beschriebenen Bioassays geprüft. Die aktiven Fraktionen werden sodann nochmals auf ihre Reinheit überprüft um sie danach der Strukturaufklärung zuzuführen.

Schritt 5: In einem weiteren Verfahrensschritt wird die exakte chemische Struktur der biologisch aktiven Substanzen bestimmt Solche Analysen (A) erfolgen analog zur Strukturaufklärung in der Naturstoffchemie und lassen sich mit bekannten und bewährten Analysemethoden wie beispielsweise HPLC, GC, Massenspektrometrie, NMR, Elementaranalyse, UV- und IR-Spektroskopie, Röntgenkristallspektroskopie, etc, bewerkstelligen. Das Resultat dieser Analysen ist eine unweideutige Molekülstruktur, aus deren Kenntnis der Fachmann mittels bekannter Methoden die Substanz chemischsynthetisch herstellen kann.

Die Verfahrensschritte umfassen somit, in Kurzform beschrieben:
1) Kombinatorisches Mischen eines Cocktails C mit bekannten Edukten,
2) Zufallsreaktion des Cocktails C zu einem Produkte-Cocktail C mit unbekannten chemischen Verbindungen mittels Bestrahlung.
3) Ansprechen der unbekannten Produkte des Cocktails C auf spezifische biologische Testsysteme B,
4) gegebenenfalls Isolierung und Reindarstellung von aktiven Komponenten des Coktails C.
5) Strukturaufklärung dieser unbekannten Produkte.

## Patentansprüche

1. Verfahren zur Zufallserzeugung und Prüfen chemischer Verbindungen durch folgende Verfahrensschritte:
kombinatorisches Herstellen von Mischungen aus bekannten, löslichen Edukten;
Bestrahlen der Mischungen, derart, dass die bekannten Edukte der Mischungen reagieren und Produkte-Cocktails mit unvorhersehbaren Zusammensetzungen entstehen;
Testen der Produkte-Cocktails in spezifischen, biologischen Testsystemen auf spezifische, biologische Aktivitäten;
Auftrennen des Aktivität aufweisenden Produktecocktails mit nicht destruktiven Methoden und Prüfen der einzelnen Fraktionen hinsichtlich biologischer Aktivität.

2. Verfahren nach Anspruch 1, **dadurch gekennzeichnet, dass** die Struktur der aktiven Komponenten aufgeklärt wird.

3. Verfahren nach Anspruch 1 oder 2, **dadurch gekennzeichnet, dass** die Bestrahlung eine ionisierende Strahlung oder ein Neutronenbeschuss ist.

## Claims

1. Method for the random generation and testing of chemical compounds by means of the following method steps:
Combinatorial production of mixtures from known, soluble educts;
exposure of mixtures to radiation, such that the known educts react to become product cocktails of unpredictable composition;
testing of the product cocktails in specific biological test systems for specific biological activities;
separation of the product cocktails showing activity by means of non-destructive methods and testing of the individual fractions concerning biological activity.

2. Method according to claim 1, **characterized in that**, the structure of the isolated components is determined.

3. Method according to claim 1 or 2, **characterized in that** the exposure to radiation is effected by means of an ionizing radiation or neutron bombardment.

## Revendications

1. Procédé de création aléatoire et de vérification de composés chimique qui comprend les étapes:
préparer de manière combinatoire des mélanges de réactifs connus et solubles,
irradier les mélanges de telle sorte que les réactifs connus des mélanges réagissent et fournissent des cocktails de produits de composition imprévisible,
tester les activités biologiques spécifiques du cocktail de produits dans des systèmes biologiques spécifiques de test,
séparer le cocktail de produits qui présente une activité par des méthodes non destructives et vérification de l'activité biologique des fractions individuelles.

2. Procédé selon la revendication 1, **caractérisé en ce que** l'on détermine la structure des composants actifs.

3. Procédé selon la revendication 1 ou 2, **caractérisé en ce que** le rayonnement d'irradiation est un rayonnement ionisant ou un bombardement par neutrons.
